# NEUE EUROPÄISCHE PATENTSCHRIFT

(11) **EP 0 593 901 B2**
(45) Veröffentlichungstag und Bekanntmachung des Hinweises auf die Entscheidung über den Einspruch: **09.04.2008**
(45) Hinweis auf die Patenterteilung: 16.04.1997
(21) Anmeldenummer: 93114773.0
(22) Anmeldetag: 14.09.1993
(51) Int. Cl.: C07H 21/00, A61K 31/70, C12N 15/11, C12N 9/00

(54) **Oligoribonucleotid- und Ribozym-Analoga mit terminalen 3'-3'-bzw.5'-5'-Verknüpfungen**
Oligoribonucleotide- and ribozyme-analoguer with terminal 3',3'- and 5',5'-bonds respectively
Analogues des oligoribonucleotide et ribozymer avec 3'-3'-et 5'-5' liasons terminaler

(30) Priorität: 24.09.1992 DE 4231949
(43) Veröffentlichungstag der Anmeldung: 27.04.1994
(73) Patentinhaber: Sanofi-Aventis Deutschland GmbH, 65929 Frankfurt am Main (DE)
(72) Erfinder: Seliger, H., Prof. Dr., d-89275 Elchingen (DE); Ortigao, Flavio Ramalho, Dr., D-89075 Ulm (DE); Rösch, Hannelore, D-89155 Erbach (DE); Rösch, Rudi, D-89155 Erbach (DE); Krist, Bernd, D-89075 Ulm (DE)

(56) Entgegenhaltungen:
- EP-A- 0 399 330
- EP-A- 0 464 638
- EP-A- 0 552 766
- EP-A- 0 552 767
- WO-A-92/13869
- CHEMICAL REVIEWS Bd. 90, Nr. 4 , 1990 Seiten 544 - 584 E.UHLMANN ET AL. 'Antisense Oligonucleotides: A New Therapeutic Principle.'
- TRENDS IN BIOTECHNOLOGY Bd. 10, Nr. 5 , 1992 , CAMBRIDGE, GB Seiten 152 - 158 S.AGRAWAL ET AL. 'Antisense Oligonucleotides as Antiviral Agents.'
- NUCLEOSIDES AND NUCLEOTIDES Bd. 10, Nr. 1-3 , 1991 Seiten 469 - 477 H.SELIGER ET AL. 'Oligonucleotide Analogues with Terminal 3'-3'- and 5'-5'- Internucleotidic Linkages as Antisense Inhibitors of Viral Gene Expression'
- Antisense Research and Development, Bd. 2, 1992, Seiten 129-146, J.F.R. Ortigao et al.
- Biochemistry, Bd. 23, 1984, Seiten 6153-6159, J.G. Nadeau et al.

## Beschreibung

Die Erfindung betrifft Oligoribonucleotidanaloga mit terminalen 3'-3'- bzw. 5'5'-Internucleotidverknüpfungen. Diese Modifikation stabilisiert die so veränderten Moleküle, darunter auch Ribozyme, ohne ihre Eigenschaften, darunter auch gegebenenfalls katalytische Aktivitäten, nachteilig zu verändern.

Als antisense-Oligonucleotide bezeichnet man Nucleinsäurefragmente, deren Sequenz komplementär zur codierenden oder "sense"-Sequenz einer Messenger-RNA bzw. zum codogenen Strang der DNA ist. Solche Oligonucleotide finden in wachsendem Maße Anwendung zur Inhibition der Genexpression, meist unter medizintherapeutischen Gesichtspunkt, in vitro, in Zellkultursystemen und in vivo (1. E.Uhlmann, A.Peyman, Chem.Rev.90 (1990) 543-584; 2. J.Goodchild, Bioconjugate Chem. 1 (1990) 165-187; 3. L.Whitesell, A.Rosolen, L.Neckers, Antisense Research and Development 1 (1991) 343).

Variationen des antisense-Prinzips sind:
I. Tripelhelix-bildende Oligonucleotide: Nucleinsäurefragmente, die unter Ausbildung einer Tripelhelix an den DNA-Doppelstrang bilden können und die durch Inhibierung der Transkription die Genexpression modulieren (J.Chubb and M.Hogan, TIBTECH 10 (1992) 132-136).
II. Ribozyme: Ribonucleinsäurefragmente mit enzymatischer Aktivität, die darin besteht, daß die Ziel-RNA, z.B. eine m-RNA, nach der spezifischen Anbindung des Ribozyms von demselben gespalten wird (T.R.Cech, J.Am.Med. Assoc.260 (1988) 3030).

Damit antisense-Oligonucleotide, Tripelhelixbildende Oligonucleotide und Ribozyme in biologischen Systemen eingesetzt werden können, müssen allerdings folgende Voraussetzungen erfüllt sein (E.Uhlmann, A.Peyman, Chem. Rev. 90 (1990, 543-584):
1. sie müssen einerseits gut wasserlöslich sein, andererseits aber die lipophile Zellmembran leicht passieren,
2. sie müssen innerhalb der Zelle hinreichend abbaustabil, d.h. stabil gegen Nucleasen sein,
3. sie müssen mit intrazellulären Nucleinsäuren bei physiologischen Temperaturen stabile Hybride bilden,
4. die Hybridisierung muß selektiv sein; der Unterschied der Dissoziationstemperatur zu einem Oligonucleotid, das eine Fehlpaarung ergibt, muß hinreichend groß sein, so daß letzteres noch spezifisch abgewaschen werden kann,
5. bei Ribozymen muß die katalytische Aktivität erhalten bleiben.

Unmodifizierte Oligonucleotide und insbesondere unmodifizierte Oligoribonucleotide sind in hohem Maße nukleolytischem Abbau unterworfen. Schon früh wurden daher Untersuchungen durchgeführt, Oligonucleotide strukturell so zu modifizieren, daß sie die obengenannten Anforderungen besser erfüllen, insbesondere gegen Nuclease-Abbau besser geschützt sind. Zu diesem Zweck wurde eine große Zahl von Oligonucleotid-Analogen, teilweise unter enormem synthetischem Aufwand, hergestellt (1. E.Uhlmann, A.Peyman, Chem.Rev.90 (1990) 543-584; 2. J.Goodchild, Bioconjugate Chem. 1 (1990) 165-187).

Aus der europäischen Patentanmeldung EP 0 552 767 A2 sind 3'-derivatisierte Oligonukleotidanaloga mit nicht nukleotidischen Gruppierungen, deren Herstellung und Verwendung bekannt.

Aus der europäischen Patentanmeldung EP 0 399 330 A1 ist ein modifiziertes Phosphoramiditverfahren zur Synthese von Nukleotidsequenzen bekannt. Durch Einsatz eines modifizierten Nukleosidphosphoramidits ist es demgemäß möglich Nukleotidsequenzen herzustellen, die einen modifizierten Phosphatrest aufweisen.

S. Agrawal (TIBTECH 10: 152-158, 1992) beschreibt das Wirkprinzip von verschiedenen Typen von Antisense Oligonukleotidanaloga. Da manche Vertreter dieser Verbindungsklassen relativ Nuklease stabil sind, stellen die erhaltenen Verbindungen die Entwicklung einer neuen Klasse antiviraler Chemotherapeutika in Aussicht.

Die internationale Patentanmeldung WO 92/13869 beschreibt Methylenphosphonat Nukleosid- und daraus hergestellte Oligonukleotid-Analoga. Die beschriebenen Oligonukleotide können an der 2'-Position mit H, OH, F OMe, SMe, O-allyl, S-allyl substituiert sein.

Kürzlich wurde gezeigt, daß 3'-3'- bzw. 5'-5'-terminal verknüpfte Oligodesoxynucleotide bzw. deren Analoga eine deutlich erhöhte Stabilität gegen nucleolytischen Abbau aufweisen (1. H. Seeliger, A. Fröhlich, M. Montenarh: Nucleosides & Nucleotides 10 (1991) 469-477; 2. H. Rösch, A. Fröhlich, J. Ramalho-Ortigao, J. Flavio, M. Montenarh, H. Seeliger: EP 0 464 638 A2). Überraschend wurde nun gefunden, daß der gleiche, synthetisch leicht zugängliche, terminale Verknüpfungstypus
a) auch die sehr viel labileren Oligoribonucleotide gegen Nucleasen zu stabilisieren vermag,
b) Ribozyme (Oligoribonucleotide mit besonderer Sequenzanforderung) gegen Nucleasen zu stabilisieren vermag, ohne die katalytische Aktivität zu beeinträchtigen.
c) Oligoribonucleotide bzw. Ribozyme, die durch chemische Modifikation gegen Nucleasen geschützt wurden, zusätzlich zu stabilisieren vermag.

Die Erfindung betrifft daher Oligoribonucleotide der Formel I 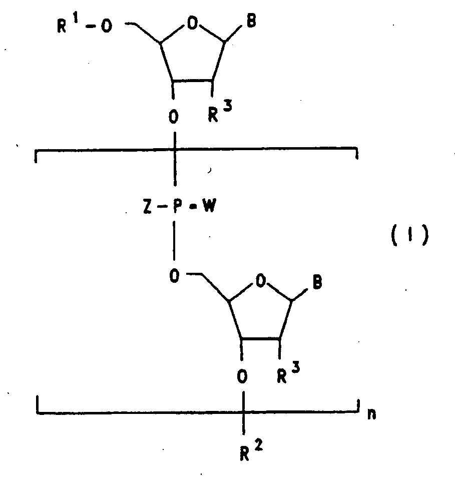 worin
- R¹: Wasserstoff oder einen Rest der Formel II bedeutet 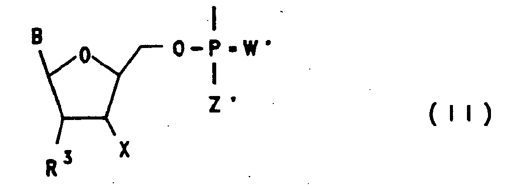
- R²: einen Rest der Formel III bedeutet,

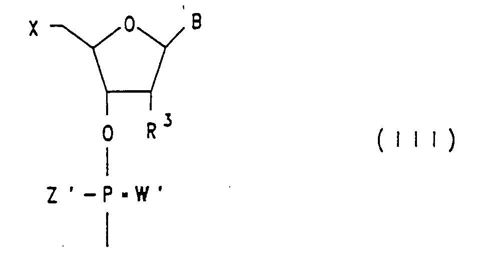

B für eine Base, wie natürliche Basen wie Adenin, Thymin, Cytosin, Guanin oder unnatürliche Basen, wie Purin, 2,6-Diaminopurin, 7-Deazaadenin, 7-Deazaguanin, N⁴, N⁴-Ethanacytosin bzw. deren Prodrugformen steht; wobei die Base Uracil oder Cytosin im Oligoribonukleotid enthalten ist;
R³ unabhängig voneinander OH, Wasserstoff oder F bedeutet, höchstens ein
R³-Rest gleich H ist, und entweder bei allen U-Nukleosiden oder bei sowohl allen U-als auch C-Nukleosiden R³ gleich F ist;

- W und W': unabhängig voneinander Sauerstoff oder Schwefel bedeutet;
- Z und Z': unabhängig voneinander O⁻; S⁻; C₁-C₁₈-Alkoxy, bevorzugt C₁-C₈-Alkoxy, C₁-C₁₈-Alkyl, bevorzugt C₁-C₈-Alkyl, NHR⁴, mit R⁴ = C₁-C₁₈-Alkyl, oder C₁-C₄-Alkoxy-C₁-C₆-Alkyl, bevorzugt Methoxyethyl; NR⁴R⁵, worin R⁴ wie oben definiert ist und R⁵ C₁-C₁₈-Alkyl, bevorzugt C₁-C₈-Alkyl bedeutet oder worin R⁴ und R⁵ zusammen mit dem sie tragenden Stickstoffatom einen 5-6-gliedrigen heterozyklischen Ring bedeutet, der zusätzlich ein weiteres Heteroatom aus der Reihe O, S, N enthalten kann, wie z.B. Morpholin;
- wobei X steht für: OH, H, F, Cl, Br, NH₂, N₃, O-C(O)-(C₁-C₁₈)-Alkyl, O-C(O)-(C₂-C₁₈)-Alkenyl, O-C(O)-(C₂-C₁₈)Alkinyl, O-C(O)-(C₆-C₁₈)Aryl, O-(C₁-C₁₈)-Alkyl, O-(C₂-C₁₈)-Alkenyl, O-(C₂-C₁₈)Alkinyl, O-(C₆-C₁₈)Aryl, P(O)YY', wobei Y und Y' wie Z und Z' definiert sind. In Formel II können R3 und X zusammen einen cyclischen Phosphorsäurediester bilden.
Bevorzugt steht X für OH, H, F;
und wobei
- n: eine ganze Zahl von 5-60, bevorzugt 15-25 bedeutet,
sowie deren physiologisch verträglichen Salze.

Unter Aryl soll in diesem Zusammenhang verstanden werden z.B. Phenyl, Phenyl substituiert (1-3 fach) mit C₁-C₆-Alkyl, C₁-C₆-Alkoxy und/oder Halogen.

Bevorzugt sind die Oligoribonucleotide der Formel I, wobei R³ bei allen U- und C-Nucleosiden gleich F ist. Ferner sind Oligoribonucleotide der Formel I bevorzugt, worin R² für einen Rest der Formel III steht und R¹ Wasserstoff bedeutet; R¹ bzw. R² für einen Rest der Formeln II bzw. III steht; wobei entweder W oder Z im letzten Fall nicht Sauerstoff bedeuten und X für OH oder H steht.

Insbesondere seien ferner Oligoribonucleotide der Formel I genannt, worin W für Sauerstoff steht oder Z und W beide für Sauerstoff stehen.

Insbesondere seien ferner Oligoribonucleotide der Formel I genannt, deren Basenfolge B¹, B²,.....Bⁿ den Sequenzanforderungen für Ribozyme entspricht.

Hierbei sollen "Hammerhead-Ribozyme" (z.B. Uhlenbeck, Nature 328 (1987) 596; Haseloff, Gerlach, Nature 334 (1988) 585), die "Hairpin-Ribozyme", (z.B. Hampel et al., Nucl. Acids. Res. 18 (1990) 299) das "Human Hepatitis α-Virus-Ribozym" (z.B.Branch, Robertson, Proc. Natl.Acad.Sci. USA 88 (1991) 10163) und die "external guide sequence für RNase P" (z.B. Forster, Altman, Science 249 (1990) 783) hervorgehoben werden, ganz besonders jedoch die "Hammerhead-Ribozyme".

Ganz besonders bevorzugt sind Oligoribonucleotide der Formel I, worin R² für einen Rest der Formel III steht und R¹ Wasserstoff bedeutet.
Ferner seien Oligoribonucleotide der Formel I genannt, die zusätzlich durch Gruppen substituiert sind, die die intrazelluläre Aufnahme begünstigen, die in vitro oder in vivo als Reportergruppen dienen, und/oder Gruppen, die bei der Hybridisierung des Oligoribonucleotids an biologische DNA oder RNA diese DNA- oder RNA Moleküle unter Bindung oder Spaltung angreifen.

Beispielhaft für Gruppen, die die intrazelluläre Aufnahme begünstigen, sind lipophile Reste wie Alkylreste z.B. mit bis zu 18 C-Atomen oder Cholesteryl, oder Thiocholesteryl (E.Uhlmann, A.Peyman, Chem.Rev.90 (1990) 543-584; J.Goodchild, Bioconjugate Chem. 1 (1990) 165-187; B.Oberhauser, E.Wagner, Nucl.Acids Res. 20 (1992) 533; C.MacKellar et al. Nucl.Acids Res. 20 (1992) 3411) oder Konjugate, die natürliche Carrier-Systeme ausnutzen, wie z.B. Gallensäure oder Peptide für den entsprechenden Rezeptor (z.B. rezeptorvermittelte Endozytose).
Beispiele für Reportergruppen sind fluoreszierende Gruppen (z.B. Acridinyl, Dansyl, Fluoresceinyl) oder chemilumineszierende Gruppen wie z.B. Acridiniumester-Gruppen.

Beispiele für Oligonucleotid-Konjugate, die an Nucleinsäuren binden und/oder spalten, finden sich in nachstehenden Zitaten. (E.Uhlmann, A.Peyman, Chem.Rev.90 (1990) 543-584; J.Goodchild, Bioconjugate Chem. 1 (1990) 165-187; Helene, Toulme, Biochim. Biophys, Acta 1049 (1990) 99).
Konjugatpartner sind u.a. Acridin, Psoralen, Chlorethylaminoaryl, Phenanthridin, Azidophenazyl, Azidoproflavin, Phenazin, Phenanthrolin/Cu, Porphyrin/Fe, Benzo[e]pyridoindol, EDTA/Fe (Mergny et al, Science 256 (1992) 1681).

Die charakteristische strukturelle Modifikation der erfindungsgemäßen Oligoribonucleotide besteht darin, daß die Internucleotidverknüpfungen an den beiden Kettenenden verändert sind, d.h. statt biologischer 3'-5'-Verknüpfungen 3'-3'-bzw. 5'-5'-Verknüpfungen sind. Überraschenderweise wurde gefunden, daß diese minimale Strukturmodifikation genügt, um solche Verbindungen gegen Nuclease-Abbau zu stabilisieren, ohne andere Eigenschaften, z.B. enzymatische Aktivitäten nachteilig zu verändern.

Wie nachstehend beschrieben wird, bewirkt die nur geringfügige Strukturmodifikation ein Hybridisierungsverhalten, das fast dem der biologischen Oligoribonucleotide gleicht. Hieraus resultiert auch die allgemeine Anwendbarkeit dieser Verbindungen als Inhibitoren der Genexpression.

Die Darstellung der Verbindungen der Formel I erfolgt in gleicher Weise wie die Synthese biologischer Oligonucleotide in Lösung oder vorzugsweise an fester Phase, gegebenenfalls unter Zuhilfenahme eines automatischen Synthesegeräts. Gegenstand der Erfindung ist daher weiterhin ein Verfahren zur Herstellung der Oligoribonucleotide der Formel I, dadurch gekennzeichnet, daß man
a) eine Nucleotideinheit mit 3'-bzw. 5'-terminaler Phosphor(III)- oder Phosphor(V)-Gruppierungen oder deren aktiviertes Derivat mit einer weiteren Nucleotideinheit mit 3'- bzw. 5'-terminaler freier Hydroxygruppe umsetzt oder
b) das Oligonucleotid durch Fragmente in gleicher Weise aufbaut, in den nach (a) oder (b) erhaltenen Oligonucleotiden gegebenenfalls eine oder mehrere zum Schutz anderer Funktionen temporär eingeführte Schutzgruppen abspaltet und die so erhaltenen Oligonucleotide der Formel I gegebenenfalls in ihr physiologisch verträgliches Salz überführt.

Als Ausgangskomponente zur Herstellung von Oligoribonucleotiden mit terminal invertierter 3'3' Bindung wird für die Festphasensynthese ein Trägerharz eingesetzt, an welches das erste Nucleosidmonomere über die 5'-OH-Gruppe angeknüpft ist. Zur Herstellung dieser Komponente verwendet man ein nach literaturbekannten Methoden (T. Atkinson, M Smith in Oligonucleotide Synthesis, M.J. Gait (ed), 35-49 (1984)) dargestelltes Trägerharz, vorzugsweise Kieselgel oder "controlled pore glass", das mit Aminogruppen funktionalisiert ist. Es wird mit einem an der Nucleobase und an der 3'-OH Gruppe geschützten Nucleosidderivat umgesetzt, das zuvor ins 5'-p-Nitro-phenylsuccinat umgewandelt worden war. Als Basenschutzgruppen werden vorzugsweise Acylgruppen, z.B. Benzoyl, Isobutyryl oder Phenoxyacetyl eingesetzt. Die 3'-Position wird vorzugsweise durch die Dimethoxytrityl-Schutzgruppe geschützt, die nach M.D.Matteucci, M.H.Caruthers, Tetrahedron Letters 21 (1980) S.3243-3246, eingeführt werden kann.

Der weitere Aufbau der Oligoribonucleotidkette bis zum vorletzten Kettenglied erfolgt nach literaturbekannten Methoden (Beaucage, lyer, Tetrahedron 48 (1992) 2223), vorzugsweise unter Verwendung von an der 5'-OH Gruppe durch Dimethoxytritylgruppen geschützten Nucleosid-3'-phosphorigsäureesteramiden oder Nucleosid-3'-H-phosphonaten. Die 2'-Hydroxygruppe wird vorzugsweise durch die tert.-Butyldimethylsilylgruppe geschützt. (M.Lytttle et al., J.Org.Chem. 56 (1991) 4608; Scaringe et al., Nucl. Acids Res. 18 (1990) 5433). Als letztes Kettenglied wird wieder ein an der 3'-OH-Gruppe, vorzugsweise mit Dimethoxytrityl geschützes Nucleosid-5'-phosphorigsäureesteramid bzw. Nucleosid-H-phosphonat eingesetzt. Die Darstellung einer solchen Oligoribonucleotidkette mit terminal verdrehten Internucleotidbindungen ist nachfolgend schematisch wiedergegeben. (Phosphoramiditzyklus zur Darstellung von Oligonucleotiden mit 3'-3'- und 5'-5'-Verknüpfungen an den Enden). Die Darstellung von Oligoribonucleotiden mit 3'-3'- oder 5'-5'-Verknüpfungen erfolgt entsprechend.

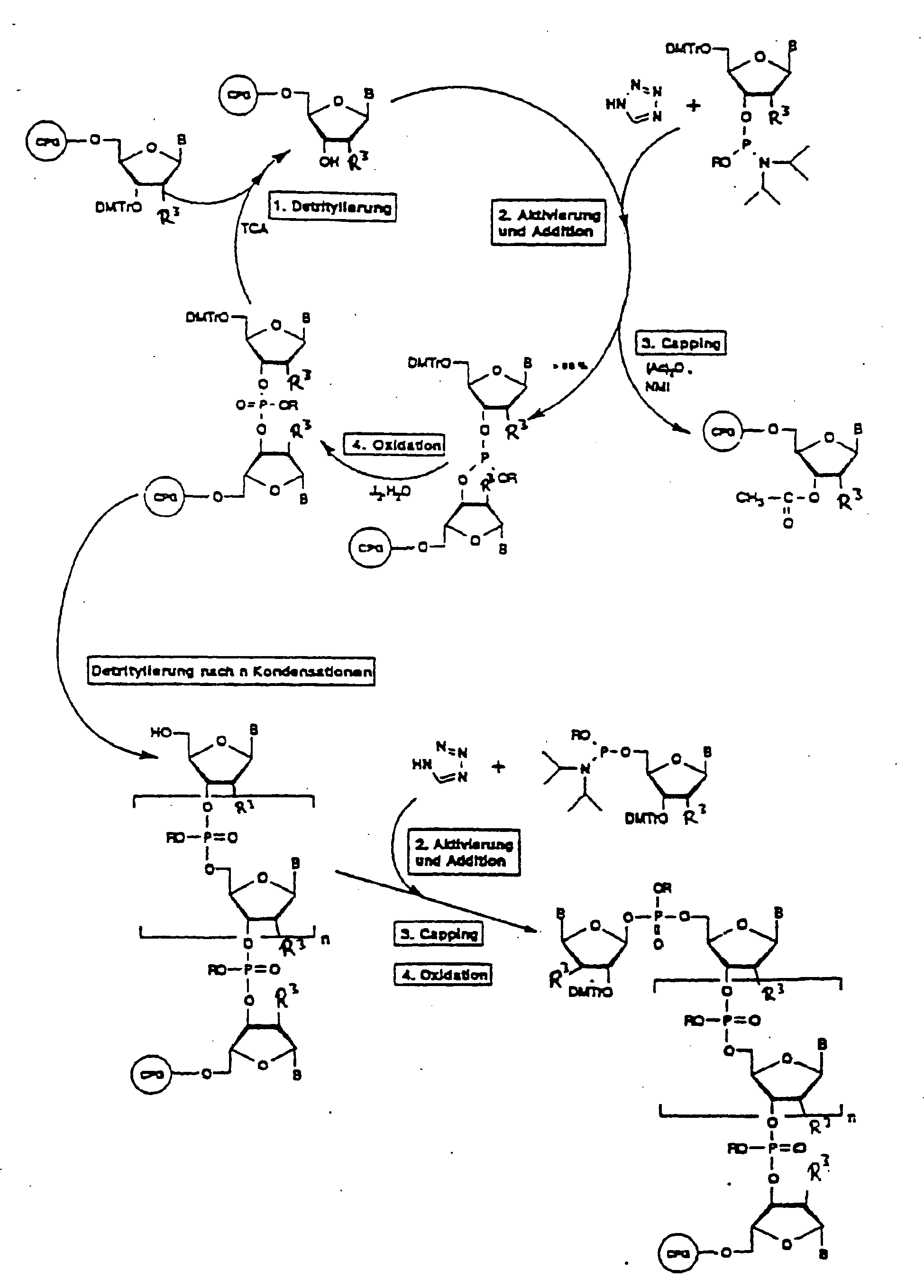

Ebenfalls nach literaturbekannten Methoden erfolgt der Einbau 2'-modifizierter Ribonucleotideinheiten wie z.B. 2'-O-Alkyl 2'-deoxyribonucleotiden (Iribarren et al., Proc. Natl. Acad. Sci. USA 87 (1990) 7747; Sproat, Lamond in Oligonucleotides and Analogues: F.Eckstein, Ed., IRL Press, Ocford 1991); 2'-F-2'deoxyribonucleotiden (Benseler et al., Nucleosides & Nucleotides 11 (1992) 1333; Pieken et al., Science 253 (1991) 314; Olsen et al., Biochemistry 30 (1991) 9735).

Für Struktur- und Sequenzanalysen werden die Oligoribonucleotide terminal markiert, wie nachstehend in Beispiel 4 beschrieben. Dies geschieht durch radioaktive Markierung, vorzugsweise mit Hilfe von 5'-γ³²P-ATP/Polynucleotidkinase. Diese radioaktive Markierung erfolgt an der freien 5'-OH-Gruppe, d.h. gegenüber einem Oligonucleotid mit nur biologischem 3'-5'-Verknüpfungen am umgekehrten Ende der Nucleotidkette.

Die Sequenzen mit einer 3'-3'-Invertierung besitzen beidseitig eine 5'-OH-Gruppe und werden daher teilweise an beiden Seiten phosphoryliert.

Die Oligonucleotide der Formel I finden Anwendung für hybridisierungschemische, auf der Anlagerung an doppel- oder einzelsträngige Nucleinsäuren bzw. deren Spaltung beruhende Verfahren der Regulation oder Unterdrückung der biologischen Funktion von Nucleinsäuren sowie zur selektiven Unterdrückung der Expression viraler Genomfunktionen und zur Prophylaxe und Therapie von Virusfunktionen, zur Unterdrückung der Onkogenfunktion und zur Therapie von Krebserkrankungen.

Als Maß für die Stabilität in vivo kann das Verhalten eines im Blutserum gelösten erfindungsgemäß aufgebauten Oligoribonucleotids der Formel I angesehen werden. Der allgemeine Test ist in Beispiel 4 beschrieben. Die erfindungsgemäßen Oligoribonucleotide werden im Gegensatz zu den 3'-5'-Oligoribonucleotiden viel langsamer abgebaut.

In Beispiel 5 wird demonstriert, daß die erfindungsgemäßen Oligoribonucleotide, die den Sequenzanforderungen für Hammerhead-Ribozyme genügen, sich in ihrer enzymatischen Aktivität nicht von den unmodifizierten Ribozymen unterscheiden.

Beschreibung der Figuren:
- Fig. 1:: Stabilität von p53-INV in Serum
- Fig. 2:: Kinetik der Substratspaltung durch modifizierte Ribozyme
- Fig. 3:: Nucleolytischer Abbau von p53-1 und p53-INV
- Fig. 4:: Nucleolytischer Abbau von Fp 53- INV und p53-F(U,C),INV

### Beispiel 1: (präparatives Beispiel)

### Synthese der 2'-Fluor-2'desoxynucleosideinheiten (Anhang 1) 5'-O-(Dimethoxytrityl)-2'-fluor-2'-desoxyuridin

In einem 50 ml Schlenkkolben werden 0,5 g (ca. 2 mMol) 2'-Fluor-2'-desoxyuridin mit zwei mal 10 ml abs. Pyridin coevaporiert. Das getrocknete Nucleosid wird mit 25 ml abs. Pyridin aufgenommen und bei Raumtemperatur mit 0,66 g (ca.2,2 mMol) Dimethoxytritylchlorid und 10 mg 4-Dimethylaminopyridin versetzt. Nach drei Stunden wurde dem Ansatz 1 ml Methanol zugesetzt und danach im Vakuum bis zur Trockne eingeengt. Das zurückbleibende Öl wird in 50 ml Methylenchlorid (über Aluminiumoxid entsäuert) aufgenommen und drei mal mit 50 ml Wasser extrahiert. Die organische Phase trocknet man über Natriumsulfat. Nach dem Abziehen des Methylenchlorids blieb das Rohprodukt als fester Schaum zurück. Zur Entfernung höher tritylierter Bestandteile und des Tritanols wurde das Rohprodukt bei 40-50°C mit 30 ml abs. Benzol digeriert.
Man erhält 0.75 g entsprechend 67% der Theorie eines weißen Feststoffs.

### 4-N-Acetyl-2'-fluor-2'-desoxycytidin

In einem 100 ml Schlenkkolben wird 1 g (ca. 4 mMol) 2'-Fluor-2'-desoxycytidin-Hydrochlorid zwei mal mit 20 ml abs. Pyridin und zwei mal mit je 10 ml abs. Acetonitril coevaporiert. Man suspendiert das getrocknete nucleosidische Material in 40 ml abs. DMF und gibt 0,6 ml (ca.4,4 mMol) Acetanhydrid zu. Im Verlauf von einem Tag tropft man 0.5 ml (4,4 mMol) abs. Triethylamin dem Ansatz zu. Das Lösungsmittel wird anschließend im Ölpumpenvakuum abgezogen. Das Rohprodukt wäscht man mit 50 ml Dieethylether und trocknet es anschließend. Die Reinigung erfolgte säulenchromatographisch (Kieselgel 60H, Säule 4 x 10 cm, Laufmittel Methylenchlorid mit 0.1% Pyridin, Gradient Methanol); das Produkt wird bei 8% Methanol eluiert.
Beim Abziehen des Lösungsmittels bleiben 0.83 g (71% der theor. Ausbeute) produkt zurück.

### 5'-O-(Dimethoxytrityl)-4-N-acetyl-2'-fluor-2'-desoxycytidin

In einem 100 ml Schlenkkolben werden 4 mMol des 4-N-Acetyl-2'-fluor-2'-desoxycytidin mit 25 ml abs. Pyridin aufgenommen und bei Raumtemperatur mit 1,3g (ca. 4,4 mMol) Dimethoxytritylchlorid und 20 mg 4-Dimethylaminopyridin versetzt. Nach drei Stunden wurde dem Ansatz 1 ml Methanol zugesetzt und danach im Vakuum bis zur Trockne eingeengt. Das zurückbleibende Öl wird in 50ml Methylenchlorid (über Aluminiumoxid entsäuert) aufgenommen und drei mal mit 50 ml Wasser extrahiert. Die organische Phase trocknet man über Natriumsulfat. Nach dem Abziehen des Methylenchlorids blieb das Rohprodukt als fester Schaum zurück. Zur Aufreinigung des Rohproduktes wird an Kieselgel 60H chromatographiert (Säule 2 x 20 cm, Laufmittel Methylenchlorid mit 0.1% Pyridin, Gradient Methanol). Das Produkt wurde mit 3% Methanol in Methylenchlorid eluiert. Nach dem Abziehen des Lösungsmittels bleiben 1.39 g (59% der Theorie) eines weißen schaumigen Feststoffs zurück.

### Phosphorigsäureesteramide der 2'-Fluor-2'-desoxynucleoside

1 mMol des geschützten Monomeren wird in 5 ml abs. Methylenchlorid und 1 ml abs. Diisopropylamin gelöst. Unter Argon tropft man mit einer Einwegspritze 1,2 mMol Chloro-N,N-diisopropylamino-β-cyanoethoxyphosphin zu. Nach einer Stunde ist der Umsatz nahezu quantitativ und man kann die reaktion mit 0,1 ml Methanol abbrechen. Der Ansatz wird in 20 ml Essigsäureethylester aufgenommen und dreimal mit je 20 ml gesättigter NaCl-Lösung extrahiert. Die organische Phase wird über Natriumsulfat getrocknet und das Lösungsmittel abgezogen. Das Rohprodukt wird in 5 ml Methylenchlorid aufgenommen und aus 400 ml abs. Petrolether bei Raumtemperatur ausgefällt. Nach dem Abfritten des Niederschlags wird er an der Ölpumpe getrocknet und bei -20°C gelagert.

### 5'-O-(Dimethoxytrityl)-4-N-acetyl-2'-fluor-2'-desoxycytidin-diisopropylamino-β-cyanoethoxyphosphin:

Ansatz: 0,59 g (1 mMol) 5'-O-(Dimethoxytrityl)-4-N-acetyl-2'-fluor-2'-desoxycytidin
Ausbeute: 0,60 g (0.78 mMol, 78% d.Th.)

### 5'-O-(Dimethoxytrityl)-2'-fluor-2'-desoxyuridin-diisopropylamino-β-cyanoethoxyphosphin:

Ansatz: 0,55 g (1 mMol) 5'-O-(Dimethoxytrityl)-2'-fluor-2'-desoxyuridin
Ausbeute: 0,61 g (0.83 mMol, 83% d.Th.)

### Beispiel 2: (präparatives Beispiel)

### Beladung von CPG 10-1400-Trägermaterial mit 3'-O-Dimethoxytrityldesoxyribonucleosideinheiten (Anhang 2)

### 3'-O-DMTr-desoxyribonucleosid-5'-O-succinat

| | |
|---|---|
| Ansatz: | 1,0 mMol 3'-O-DMTr-dN |
| | 0,8 mMol Bernsteinsäureanhydrid(80 mg) |
| | 0,5 mMol Dimethylaminopyridin (61 mg) |

Die Reaktion von Bernsteinsäureanhydrid mit der 5'-OH-Gruppe der Desoxyribonucleoside wurde jeweils in 5 ml absolutem Pyridin mit DMAP als Katalysator über Nacht bei Raumtemperatur durchgeführt. Nach vollständiger Umsetzung wurde die Lösung eingeengt und das Pyridin durch 3malige azeotrope Destillation mit Toluol entfernt. Der Rückstand wurde in Dichlormethan aufgenommen, mit 10 %iger eiskalter Citronensäurelösung und H₂O gewaschen und die organische Phase im Vakuum einrotiert. Das Rohprodukt wurde in ca. 3 ml Toluol gelöst und in 200 ml n-Hexan ausgefällt.

### Trägerbeladung

| | |
|---|---|
| Ansatz: | 0,8 mMol 3'-O-DMTr-dN-5'-O-succinat |
| | 0,8 mMol p-Nitrophenol (112 mg) |
| | 2,0 mMol Dicyclohexylcarbodiimid |
| | 3 g aminopropyliertes CPG 10-1400 |

Das geschütze succinylierte Desoxytribonucleosid wurde zu einer Lösung von p-Nitrophenol in 5 ml absolutem Dioxan und 0,2 ml Pyridin gegeben und anschließend DCCI als Kondensationsmittel zugefügt. Nach 3 Stunden war die Umsetzung vollständig. Der ausgefallene Dicyclohexylharnstoff wurde unter Argon abgesaugt und das Filtrat direkt zu einer Suspension des funktionalisierten Trägermaterials in 15 ml absolutem DMF gegeben. Man fügte 0,8 ml Triethylamin hinzu und schüttelte den Ansatz über Nacht. Der beladene Träger wurde dann abgesaugt, mit Methanol und Ether gewaschen und im Exsikkator getrocknet. Zur Blockierung nicht umgesetzter Aminogruppen wurde der beladene Träger mit einer Lösung von 1 ml Essigsäureanhydrid und 50 mg Dimethylaminopyridin in 15 ml absolutem Pyridin 1 Stunde bei Raumtemperatur geschüttelt, anschließend abgesaugt, mit Methanol und Ether gewaschen und getrocknet.

### Beispiel 3:

### Synthese von Oligoribonucleotiden mit 2'-fluor-2'-desoxyuridineinheiten und einer 3'-3'-Phosphodiesterbindung am 3'-Terminus

Die Synthese des modifizierten "Hammerhead"-Ribozyms (Tab.1) wurde im 0,2 µMol Maßstab mit dem DNA-Synthesizer Gene Assember Puls der Firma Pharmacia durchgeführt. Das Trägermaterial für die Synthese war mit dem über die 5'-Hydroxylgruppe aufgehängten Desoxyadenosinbaustein funktionalisiert; in der Synthese resultiert hieraus eine invertierte Struktur am 3'-Terminus des Oligonucleotids. Die Darstellung erfolgte nach dem Standardprotokoll für Oligoribonucleotidsynthesen im Phosphorigsäureesteramidverfahren.

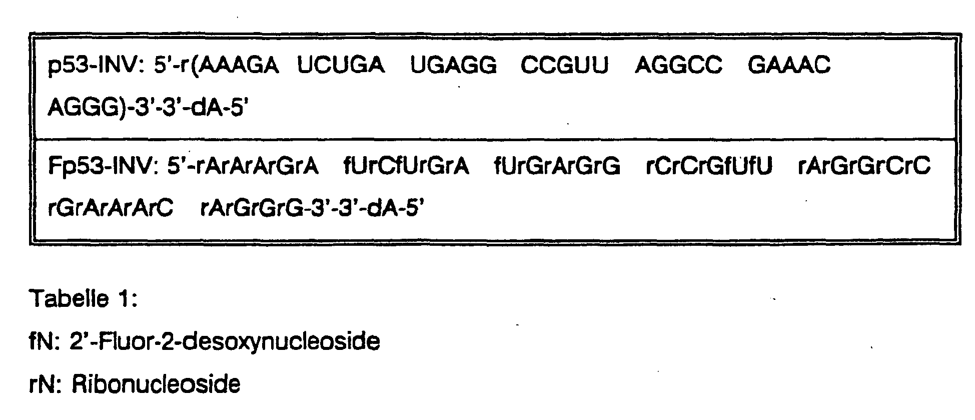

Die Phosphorigsäureesteramide der 2'-Fluor-2'-desoxynucleoside wurden 0,12 M in Acetonitril eingesetzt. Im Kettenverlängerungsschritt wird 0.1 ml des Amidophosphits mit 0.37 ml der Tetrazollösung (0,5 M) mit den trägergebundenen 5'-Hydroxylgruppen des Oligonucleotids umgesetzt. Nach einer Kopplungsdauer von 12 Minuten wird standardmäßig gekappt, oxidiert und zur Vorbereitung des nächsten Kopplungsschrittes detrityliert. Die Kopplungsausbeuten betrugen durchschnittlich 99%.

Zur Abspaltung der basenlabilen Schutzgruppen und zur Spaltung der Aufhängung wird der Träger nach der Synthese in ein verschraubbares Eppendorf-Reaktionsgefäß überführt. Bei 55°C inkubierte man 12 Stunden mit 2 ml einer 3:1 - Mischung aus 32%igem Ammoniak und Ethanol. Die überstehende Lösung wurde abgehoben, auf -20°C abgekühlt und vorsichtig lyophilisiert. Der trockene Rückstand wurde in 0,4 ml einer 1,1 M TBAF-Lösung in THF suspendiert und weitere 16 Stunden bei Raumtemperatur inkubiert. Die Reaktion wurde durch Zugabe des gleichen Volumens eines Triethylammoniumacetatpuffers (TEAA-Puffer) abgebrochen. Die Lösung wurde auf -70°C abgekühlt und vorsichtig auf 0,4 ml eingeengt. Nach der Zugabe von 40 µl Natriumacetat, 1.4 ml Ethanol und 5 µl Essigsäure wurde das Produkt über Nacht bei -20°C gefällt. Die Probe wurde zentrifugiert und der Überstand verworfen. Das trockene Oligonucleotid nahm man mit einer 1:1 Mischung aus Formamidblaumarker und Wasser aus, um es auf ein Acrylamidgel aufzugeben (20%ig, 7M Harnstoff). Um die Produktbande zu identifizieren und auszuschneiden wurde das Gel mit Cellophanfolien abgedeckt. Die Elution erfolgte bei 40°C mit Ammoniumacetatlösung. Nach 5 Stunden wurde die Lösung wie oben beschrieben gefällt. Das Oligonucleotid wurde mit 70 %igem Ethanol nachgewaschen, in 70 %igem Ethanol resuspendiert und bei -70°C gelagert.

### Beispiel 4:

### Untersuchung der Stabilität der modifizierten Ribozyme im Blutserumtest

Das Ribozym p53 sowie die modifizierten Oligoribonucleotide p53-INV und Fp53-INV wurden enzymatisch durch T4-Polynucleotidkinase in Gegenwart von ( -³²P)-dATP (spezifische Aktivität: 4500 Ci(mmol)radioaktiv markiert. Die Sequenzen mit einer 3'-3'-Invertierung besitzen beidseitig eine 5'-Hydroxygruppe und werden daher z.T. an beiden Enden phosphoryliert.

Die markierten Ribozyme wurden mit frischem menschlichem Serum behandelt.

| | |
|---|---|
| Ansatz: | 1 pMol phosphoryliertes Ribozym |
| | 20 µl Serum |

Die Proben wurden bei 37 °C inkubiert. Nach folgenden Zeiten wurden jeweils 2 µl entnommen und phenolisiert:
p53: 0, 1, 2, 5, 10, 15, 30, 60 Min.
p53-INV und Fp53-INV: 0, 1, 2, 5, 10, 15, 30, 60, 120, 240 Min.

Die phenolisierten Proben wurden lyophylisiert, in 95% Formamid - loading buffer aufgenommen und auf einem 20 %igen Polyacrylamidgel mit 8 M Harnstoff bei 55°C elektrophoretisch aufgetrennt.

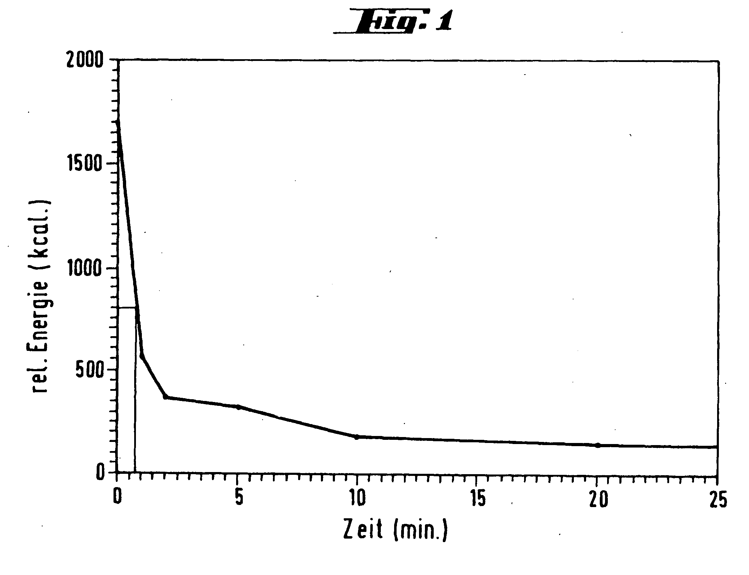

Die Intensität der Banden auf dem Röntgenfilm wurde mit Hilfe eines Laserdensitometers ermittelt (s.Fig. 1). Die Halbwertszeiten der untersuchten Ribozyme (t1/2 = 30 min für Fp53-INV, 1 min für P53-INV und < < 1 min für das biologische Oligoribonucleotid p-53 (s.Bsp.5) zeigen klar den protektiven Effekt der terminalen Invertierung.

### Beispiel 5

Ein 20-meres Substrat-Oligoribonucleotid, SB-1 5'-r(GC CCC UGU CAU CUU UUG UCC)-3' wurde enzymatisch mit T4 Polynucleotid-Kinase in Gegenwart von ³²P-ATP (spezifische Aktivität: 4500 Ci/mmol am 5'- Ende radioaktiv markiert.
Die Spaltungsreaktion von SB-1 durch verschiedene Ribozyme wurde wie folgt durchgeführt: Die Reaktionsbedingungen waren 50mM Tris HCl, pH 7,5, 20mM MgCl₂ bei 50°C. Die Substratkonzentration SB-1 betrug 0.025 µM (Variiert auf 0,05, 0,1 und 0,25 µM), die Ribozymkonzentration war 0,02 µM. Während 30 min wurde bei 1 min, 5 min, 10 min, 15 min und 30 min je eine Probe entnommen und mit loading-buffer versetzt. Die Proben wurden auf einem 20% Polyacrylamidgel (8M Harnstoff) bei 55°C elektrophoretisch aufgetrennt. Die Abnahme der Intensität der Banden von SB-1 wurde auf dem Röntgenfilm mittels eines Laserdensitometers bestimmt. Als Ribozyme wurden eingesetzt:
p53-INV; Fp53-INV (s. jew. oben); p53 und Fp53

p53: 5' -r(AAGA UCUGA UGAGG CCGUU AGGCC GAAAC AGGGA)-3'

Fp53: 5' -r(AAAGA fUCfUGA fUGAGG CCGfUfU AGGCC GAAAC AGGGA)-3'

Die Geschwindigkeit der Spaltung von SB-1 unterscheidet sich nicht beim Einsatz von p53 und p53-INV. Auch Fp53 und Fp53-INV besitzen gleiche Aktivität, die jedoch etwa um den Faktor 5 geringer ist, als die von p53.

### Beispiel 6

### Substratspaltung und kinetische Messungen

Die Messung der vorläufigen Anfangsgeschwindigkeiten der Reaktion wurde mit 40 nM Substrat und 4 nM Enzym in 50 nM Tris-Cl (pH 7,5) durchgeführt. Die Reaktion wurde durch Zugabe von 10 nM MgCl₂ gestartet. Die Menge des Spaltungsproduktes bei 55°C wurde nach 1, 2, 5, 10 und 15 Minuten gemessen.

Aus diesem Experiment wurde der Kₘ-Wert näherungsweise bestimmt. Genauere Messungen der Anfangsreaktionsgeschwindigkeiten wurden gemäß Suelter, C. H. (1985) in " A practical Guide Enzymology", J. Wiley New York, 231 durchgeführt. Hierbei wurden 40 nM Enzym in sechs getrennten Reaktionen mit jeweils 25 nM, 50 nM, 100 nM, 200 nM, 500 nM und 1000 nM Substrat durchgeführt. Nach bestimmten Zeitintervallen wurde ein Aliquot von 2 µl entnommen und die Reaktion durch Phenolzugabe gestoppt. Danach wurden die Proben auf einem denaturierenden Gel (20 % PAGE, 7 M Harnstoff) aufgetrennt und analysiert.
- Substrat:: SB-1 (Beispiel 5)
- Ribozyme:: p53-INV (Beispiel 3)

p53-F(U,C),INV: 5'-rArArArGrA fUfCfUrG
rAfUrGrArGrGfCfCrGfUfUrArGrGfC
fCrGrArArAfCrArGrGrG-3'-3'-dA-5'
p53-1: 5'-r ( AAA GAU CUG AUG AGG CCG UUA
GGC CGA AAC AGG G)-3'

### Bestimmung der kinetischen Parameter

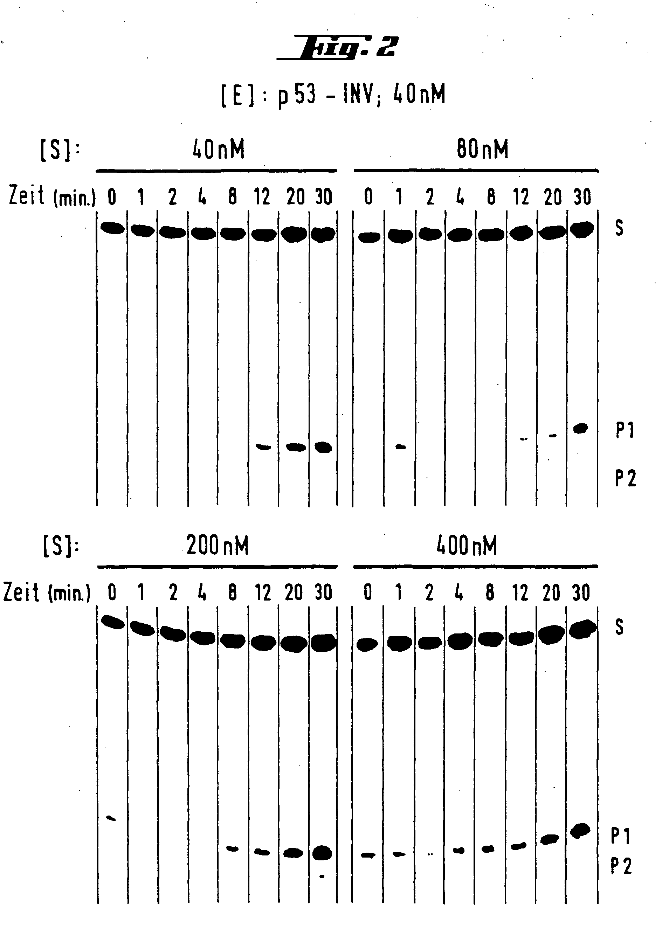

Die Anfangsreaktionsgeschwindigkeit wurde bei 5 verschiedenen Substratkonzentrationen für die frühe Phase der Progressionskurve zu dem Zeitpunkt bestimmt, als die Produktbildungsrate linear war (nach 4 Minuten). Typische Ergebnisse aus diesen kinetischen Messungen zeigt Figur 2. Da in unseren Experimenten die Reaktionen durch Zugabe von zweiwertigen Kationen zu der Reaktionsmischung gestartet wurde und die Bildung der Enzymkonformation somit nicht erlaubt wurde, wurde eine Lag-Phase gewöhnlich 5 bis 10 Minuten nach dem Reaktionsbeginn beobachtet.
Die folgenden enzymatischen Parameter wurden aus einem Eadie-Hofstee-Auftrag bestimmt.

**Tabelle 2**

| Ribozym | Kₘ (nM) | Vₘₐₓ (min⁻¹) | K_{cat} (min⁻¹) | K_{cat}/Kₘ (µM⁻¹ min⁻¹) |
|---|---|---|---|---|
| p53-1 | 24 | 8.6 | 0.21 | 8.75 |
| p53-INV | 230 | 52.3 | 1.30 | 5.65 |
| p53-F(U,C),INV | 180 | 40.9 | 1.02 | 5.56 |

### Beispiel 7

### Markierung

Substrat und Enzyme wurden ratioaktiv mit [γ-³²P]ATP und Polynukleotidkinase markiert. Nicht-eingebaute Nucleotide wurden durch Phenolextration mit anschließender Ethanol-Fällung entfernt.

### Test zur Bestimmung des Abbaus

Die Kinetik des Abbaus mit modifizierten Ribozymen wurde dadurch bestimmt, daß die radioaktiv-markierten Oligoribonukleotide in vereinigtem, frischem und nichtverdünntem Humanserum mit einer Endkonzentration von 20.000 cpm/µl gelöst wurde. Nachdem eine Anfangsprobe genommen worden war, wurde die Reaktionsmischung bei 37°C inkubiert. Nach bestimmten Zeitabschnitten wurden 1 µl Aliquote entnommen und die Reaktion mit Phenol gestoppt. Nach der Phenolextraktion und Ethanolfällung wurden die Proben in 80 % Formamid, das 20 nM EDTA, 0,01 % Bromphenolblau und 0,01 % Hylencyanot enthielt, suspendiert. Die Spaltprodukte wurden auf einem 20 %igen Polyacrylamidgel (PAGE) mit 7 M Harnstoff aufgetrennt.

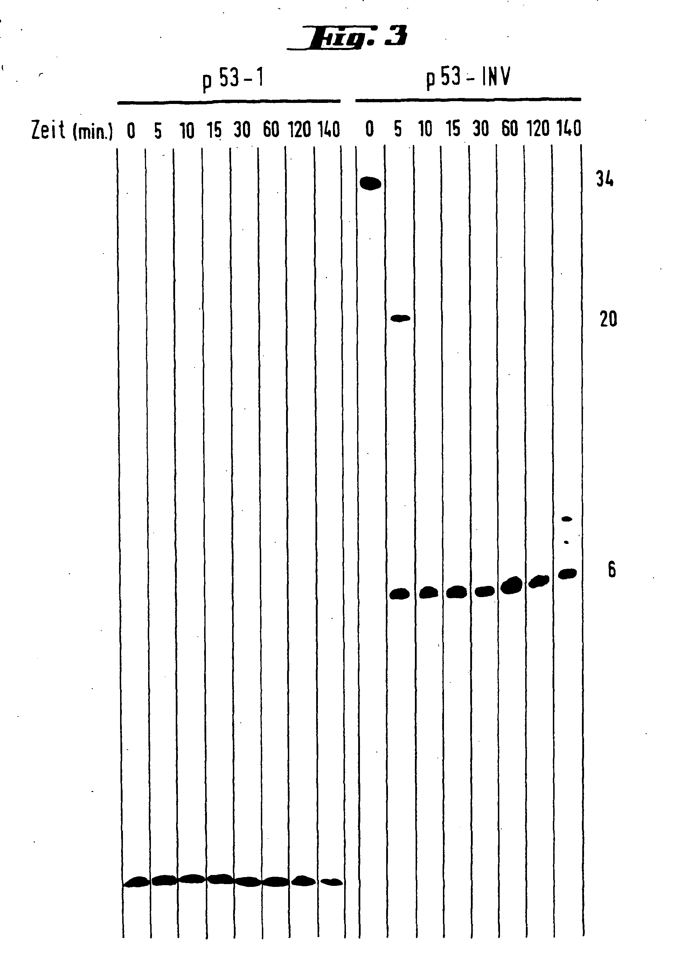

Die Ergebnisse der Inkubation von p53-1 und p53-INV sind in der Figur 3 gezeigt. Aus der Figur erkennt man, daß eine invertierte Struktur am 3'-Terminus allein bereits eine Stabilitätsverbesserung der Ribozyme in Anwesenheit von Serum von weniger als 10 Sekunden bis zu mehreren Minuten bewirkt.

Die Experimente wurden mit 1 pmol unmodifizierten (p53-1) und modifizierten (p53-INV) Ribozymen in 10 µl unverdünnten Humanserum bei 37°C durchgeführt. Die Zahlen am Gelrand entsprechen den Positionen von entsprechenden Längenstandards.

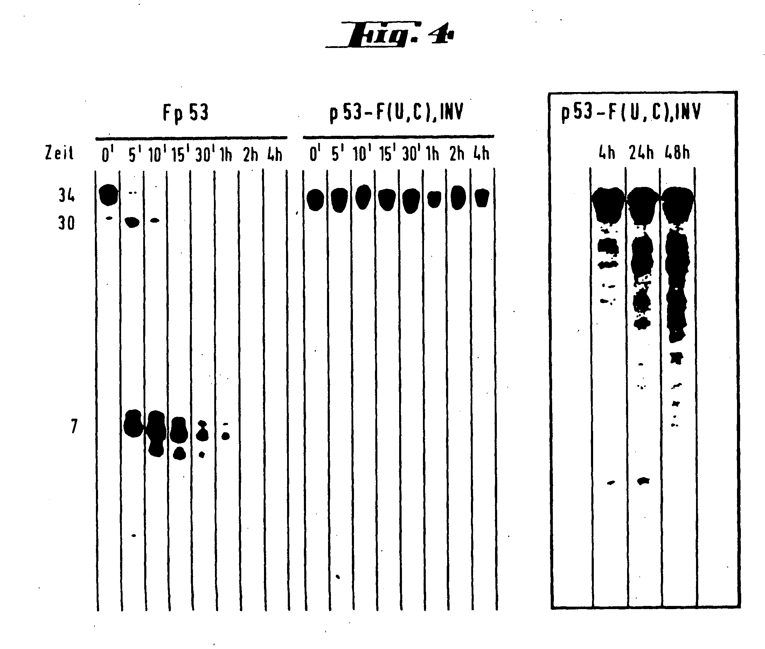

Die Ergebnisse der Inkubation von Fp53 und p53-F(U,C),INV sind in der Figur 4 gezeigt. Daraus ist zu entnehmen, daß p53-F(U,C),INV nach einer Inkubation von 4 Stunden in unverdünntem Serum kein Abbau beobachtet wurde. Nach 48 Stunden wurden weniger als 10 % Abbau beobachtet. Die Experimente wurden mit 1 pmol modifizierten Ribozymen durchgeführt, wobei Fp53 eine invertierte Struktur am 3'-Terminus besitzt und an den Positionen U₆, U₈, U₁₁, U₁₉ und U₂₀ fluoriniert ist. p53-F(U,C),INV ist zusätzlich an den Cytosinresten C₇ und C₃₀ fluoriniert.

### Anhang 1:

### Synthese von 2'-Fluor-2'desoxycytidin- (A) und 2'-Fluor-2'-desoxyuridinphosphorigsäureesteramid (B)

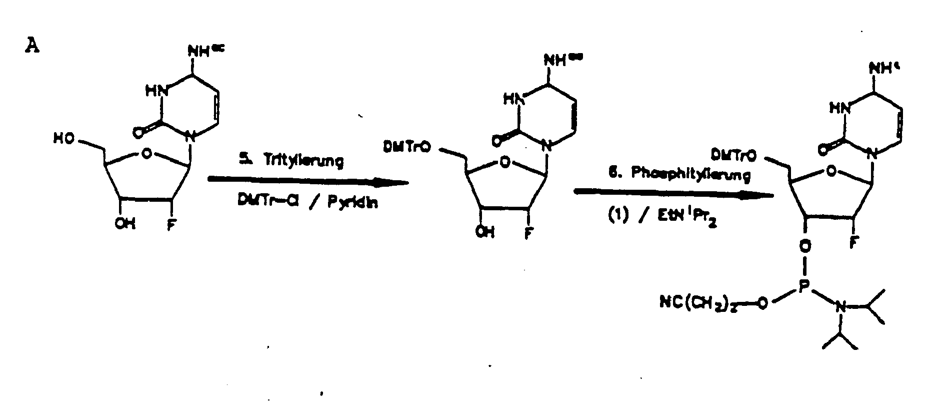

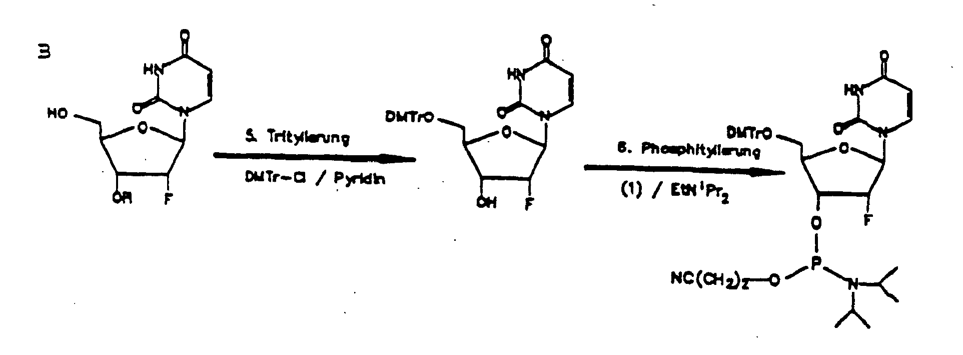

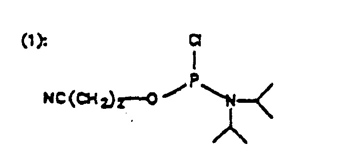

### Anhang 2:

### Synthese von 3'-O-DMTr-Desoxyribonucleosid-5'-O-succinyl-p-nitrophenylester und Belastung des Controlled - Pore - Glass Trägermaterials

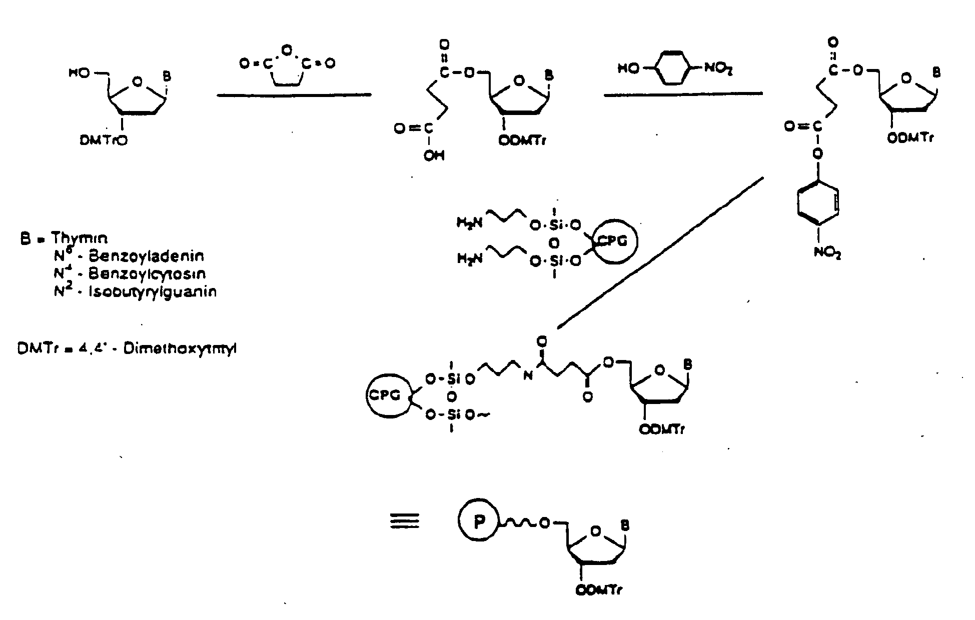

### Sequenzprotokoll

### Allgemeine Angaben

- Anmelder:: Hoechst Aktiengesellschaft
65926 Frankfurt am Main
Tel.: (069) 305-6031
Telefax: (069) 357175
- Bezeichnung der Erfindung:: Oligoribonucleotid- und Ribozym-Analoga mit terminalen 3'-3'- bzw. 5'- 5'- Verknüpfungen
Anzahl der Sequenzen: 3
Computerlesbare Fassung:
Datenträger: 3,5" HD Diskette
Computer: 386 SX
Betriebssystem: MS-DOS
Software: ASC II

### Angaben zu SEQ ID NO 1:

Sequenzkennzeichen:
- Länge:: 35 Nukleotide
- Art:: Ribonukleinsäure
- Strangform:: Einzelstrang
- Topologie:: linear
- Herkunft:: "Hammerhead"- Ribozym
- Merkmal:: invertierte Struktur am
3'-Terminus mit oder ohne
2'-Fluor-2-desoxynucleoside

### Sequenzbeschreibung: SEQ ID NO: 1

AAAGAUCUGA UGAGGCCGUU AGGCCGAAAC AGGGA

### Angaben zur SEQ ID NO 2:

Sequenzkennzeichen:
- Länge:: 34 Nukleotide
- Art:: Ribonukleinsäure
- Strangform:: Einzelstrang
- Topologie:: linear
- Herkunft:: "Hammerhead"- Ribozym
- Merkmal:: um ein Nucleotid verkürztes Ribozym

### Sequenzbeschreibung: SEQ ID NO 2:

AAGAUCUGAU GAGGCCGUUA GGCCGAAACA GGGA

### Angaben zur SEQ ID NO 3:

Sequenzkennzeichen:
- Länge:: 34 Nukleotide
- Art:: Ribonukleinsäure
- Strangform:: Einzelstrang
- Topologie:: linear
- Herkunft:: "Hammerhead"- Ribozym
- Merkmal:: um ein Nucleotid verkürztes Ribozym

### Sequenzbeschreibung: SEQ ID NO 3:

AAAGAUCUGA UGAGGCCGUU AGGCCGAAAC AGGG

## Patentansprüche

1. Oligoribonucleotide der Formel I 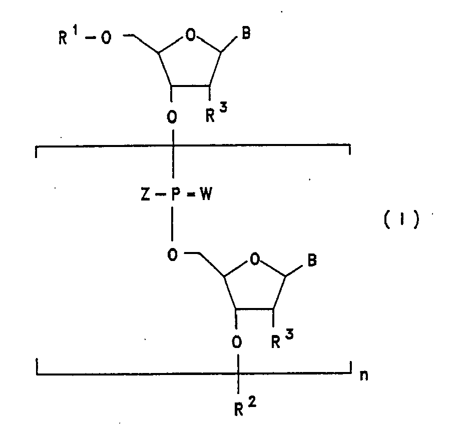 worin
R¹ Wasserstoff oder einen Rest der Formel II bedeutet 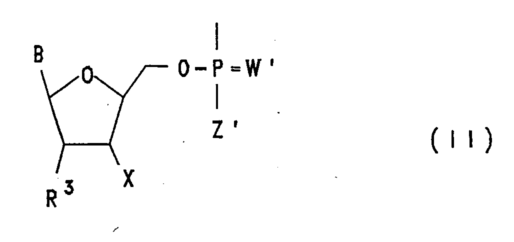
R² einen Rest der Formel III bedeutet, 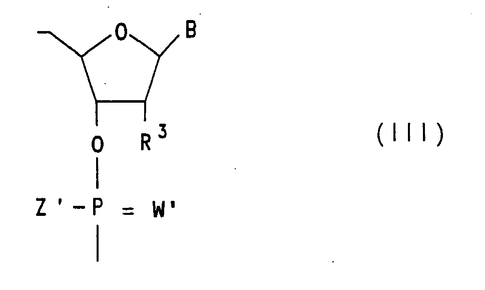
B für eine Base, wie natürliche Basen wie Adenin, Thymin, Cytosin, Guanin oder unnatürliche Basen, wie Purin, 2,6-Diaminopurin, 7-Deazaadenin, 7-Deazaguanin, N⁴, N⁴-Ethanacytosin bzw. deren Prodrugformen steht; wobei die Base Uracil oder Cytosin im Oligoribonukleotid enthalten ist;
R³ unabhängig voneinander OH, Wasserstoff oder F bedeutet, höchstens ein
R³-Rest gleich H ist, und entweder bei allen U-Nukleosiden oder bei sowohl allen U-als auch C-Nukleosiden R³ gleich F ist;
W und W' unabhängig voneinander Sauerstoff oder Schwefel bedeutet;
Z und Z' unabhängig voneinander O⁻; S⁻; C₁-C₁₈-Alkoxy, bevorzugt C₁-C₈-Alkoxy, C₁-C₁₈-Alkyl, bevorzugt C₁-C₈-Alkyl, NHR⁴, mit R⁴ = C₁-C₁₈-Alkyl, oder C₁-C₄-Alkoxy-C₁-C₆-Alkyl, bevorzugt Methoxyethyl; NR⁴R⁵, worin R⁴ wie oben definiert ist und R⁵ C₁-C₁₈-Alkyl, bevorzugt C₁-C₈-Alkyl bedeutet oder worin R⁴ und R⁵ zusammen mit dem sie tragenden Stickstoffatom einen 5-6-gliedrigen heterozyklischen Ring bedeutet, der zusätzlich ein weiteres Heteroatom aus der Reihe O, S, N enthalten kann, wie z.B. Morpholin;
wobei X steht für OH, H, F, Cl, Br, NH₂, N₃, O-C(O)-(C₁-C₁₈)-Alkyl, O-C(O)-(C₂-C₁₈)-Alkenyl, O-C(O)-(C₂-C₁₈)Alkinyl, O-C(O)-(C₆-C₁₈)Aryl, O-(C₁-C₁₈)-Alkyl, O-(C₂-C₁₈)-Alkenyl, O-(C₂-C₁₈)Alkinyl, O-(C₆-C₁₈)Aryl, P(O)YY', wobei Y und Y' wie Z und Z' definiert sind, in Formel II können R³ und X zusammen einen cyclischen Phosphorsäurediester bilden; bevorzugt steht X für OH, H, F;
n eine ganze Zahl von 5-60, bevorzugt 15-25 bedeutet,
sowie deren physiologisch verträglichen Salze.

2. Oligoribonukleotide der Formel I gemäß Anspruch 1, wobei R³ bei allen U- und C-Nukleosiden gleich F ist.

3. Oligoribonucleotide der Formel I gemäß einem der Ansprüche 1 oder 2, **dadurch gekennzeichnet, daß** R² für einen Rest der Formel III steht und R¹ Wasserstoff bedeutet; R¹ bzw. R² für einen Rest der Formeln II bzw. III steht; wobei entweder W oder Z im letzten Fall nicht Sauerstoff bedeuten und X für OH oder H steht.

4. Oligoribonucleotide der Formel I gemäß einem der Ansprüche 1 bis 3, **dadurch gekennzeichnet, daß** W für Sauerstoff steht, oder Z und W beide für Sauerstoff stehen.

5. Oligoribonucleotide der Formel I gemäß den Ansprüchen 1 bis 4, **dadurch gekennzeichnet, daß** R² für einen Rest der Formel III steht und R¹ Wasserstoff bedeutet.

6. Oligoribonucleotide der Formel I nach den Ansprüchen 1 bis 5, **dadurch gekennzeichnet, daß** diese zusätzlich durch Gruppen substituiert sind, die die intrazelluläre Aufnahme begünstigen, die in vitro oder in vivo als Reportergruppen dienen, und/oder Gruppen, die bei der Hybridisierung des Oligonucleotids an biologische DNA oder RNA diese DNA- oder RNA Moleküle unter Bindung oder Spaltung angreifen.

7. Oligoribonucleotide nach Anspruch 1, **dadurch gekennzeichnet, daß** ein Ribozym mit synthetischem 5' und/oder 3' Ende resultiert.

8. Verfahren zur Herstellung der Oligoribonucleotide der Formel I, gemäß den Ansprüchen 1 bis 6, **dadurch gekennzeichnet, daß** man
a) eine Nucleotideinheit mit 3'- bzw. 5'-terminaler Phosphor(III)- oder Phosphor(V)-Gruppierungen oder deren aktiviertes Derivat mit einer weiteren Nucleotideinheit mit 3'- bzw. 5'-terminaler freier Hydroxygruppe umsetzt oder
b) das Oligonucleotid durch Fragmente in gleicher Weise aufbaut, in den nach (a) oder (b) erhaltenen Oligonucleotiden gegebenenfalls eine oder mehrere zum Schutz anderer Funktionen temporär eingeführte Schutzgruppen abspaltet und die so erhaltenen Oligonucleotide der Formel I gegebenenfalls in ihr physiologisch verträgliches Salz überführt.

9. Oligoribonucleotide der Formel I, gemäß den Ansprüchen 1 bis 6 zur Anwendung für hybridisierungschemische, auf der Anlagerung an doppel- oder einzelsträngige Nucleinsäuren beruhende Verfahren der Regulation oder Unterdrückung der biologischen Funktion von Nucleinsäuren sowie zur selektiven Unterdrückung der Expression viraler Genomfunktionen und zur Prophylaxe und Therapie von Virusfunktionen, zur Unterdrückung der Onkogenfunktion und zur Therapie von Krebserkrankungen.

10. Arzneimittel enthaltend eine oder mehrere der Verbindungen gemäß den Ansprüchen 1-7, gegebenenfalls zusammen mit physiologisch verträglichen Hilfs- und/oder Trägerstoffen vorzugsweise für die intravenöse oder topische Verabreichung.

## Claims

1. An oligoribonucleotide of the formula I 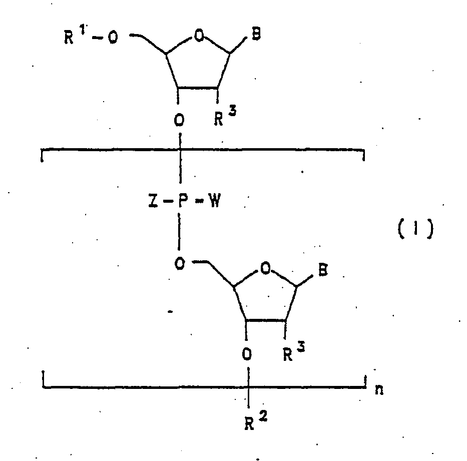 in which
R¹ is hydrogen or a radical of the formula II 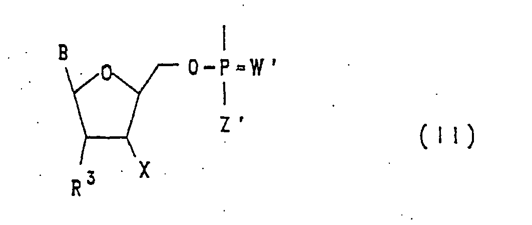
R² is a radical of the formula III 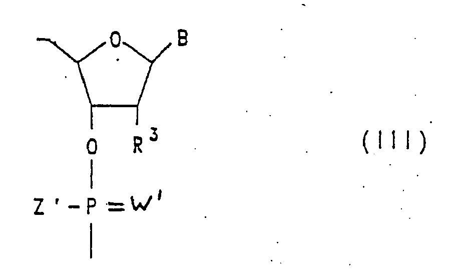
B is a base, such as natural bases such as adenine, thymine, cytosine, guanine or unnatural bases, such as purine, 2,6-diaminopurine, 7-deazaadenine, 7-deazaguanine, N⁴,N⁴-ethanacytosine or their prodrug forms; where the base uracil or cytosine is present in the oligoribonucleotide;
R³ is, independently of one another, OH, hydrogen or F, and at most one R³ radical is H, and either in all U-nucleosides or in both all U- and C-nucleosides R³ is F;
W and W' are, independently of one another, oxygen or sulfur;
Z and Z' are, independently of one another, O⁻; S⁻; C₁-C₁₈-alkoxy, preferably C₁-C₈-alkoxy, preferably C₁-C₁₈-alkyl, preferably C₁-C₈-alkyl, NHR⁴, with R⁴ = C₁-C₁₈-alkyl, or C₁-C₄-alkoxy-C₁-C₆-alkyl, preferably methoxyethyl; NR⁴R⁵, in which R⁴ is as defined above and R⁵ is C₁-C₁₈-alkyl, preferably C₁-C₈-alkyl, or in which R⁴ and R⁵ is, together with the nitrogen atom carrying them, a 5-6-membered heterocyclic ring which may additionally contain another hetero atom from the series comprising 0, S and N, such as, for example, morpholine;
where X is OH, H, F, Cl, Br, NH₂, N₃, O-C(O)-(C₁-C₁₈)-alkyl, O-C(O)-(C₂-C₁₈)-alkenyl, O-C(O)-(C₂-C₁₈)alkynyl, O-C(O)-(C₆-C₁₈)aryl, O-(C₁-C₁₈)-alkyl, O-(C₂-C₁₈)-alkenyl, O-(C₂-C₁₈)alkynyl, O-(C₆-C₁₈)aryl, P(O)YY', where Y and Y' are defined as Z and Z'; R³ and X in formula II can together form a cyclic phosphoric diester, X is preferably OH, H, F;
n is an integer from 5-60, preferably 15-25,
and its physiologically tolerated salts.

2. An oligoribonucleotide of the formula I as claimed in claim 1, where R³ in all U- and C-nucleosides is F.

3. An oligoribonucleotide of the formula I as claimed in either of claims 1 or 2, wherein R² is a radical of the formula III and R¹ is hydrogen; R¹ and R² are a radical of the formulae II and III respectively; where either W or Z in the latter case is not oxygen, and X is OH or H.

4. An oligoribonucleotide of the formula I as claimed in any of claims 1 to 3, wherein W is oxygen, or Z and W are both oxygen.

5. An oligoribonucleotide of the formula I as claimed in claims 1 to 4, wherein R² is a radical of the formula III and R¹ is hydrogen.

6. An oligoribonucleotide of the formula I as claimed in claims 1 to 5, wherein it is additionally substituted by groups which favor intracellular uptake, which act in vitro or in vivo as reporter groups, and/or groups which, on hybridization of the oligonucleotide onto biological DNA or RNA, interact with these DNA or RNA molecules with binding or cleavage.

7. An oligoribonucleotide as claimed in claim 1, wherein a ribozyme with a synthetic 5' and/or 3' end results.

8. A process for the preparation of the oligoribonucleotides of the formula I as claimed in claims 1 to 6, which comprises
a) reacting a nucleotide unit with 3' - or 5'-terminal-phosphorus(III) or phosphorus(V) groups or its activated derivative with another nucleotide unit with a 3'- or 5'-terminal free hydroxyl group, or
b) assembling the oligonucleotide by fragments in the same way, eliminating where appropriate one or more protective groups temporarily introduced to protect other functionalities in the oligonucleotides obtained according to (a) or (b), and converting the oligonucleotides of the formula I obtained in this way where appropriate into their physiologically tolerated salt.

9. An oligoribonucleotide of the formula I as claimed in claims 1 to 6 to be used for chemical hybridization methods which are based on addition onto double- or single-stranded nucleic acids for the regulation or suppression of the biological function of nucleic acids, and for the selective suppression of the expression of viral genome functions and for the prophylaxis and therapy of viral functions, for the suppression of oncogene function and for the therapy of cancers.

10. A pharmaceutical containing one or more of the compounds as claimed in claims 1-7, where appropriate together with physiologically tolerated ancillary substances and/or vehicles, preferably for intravenous or topical administration.

## Revendications

1. Oligoribonucléotides de formule I 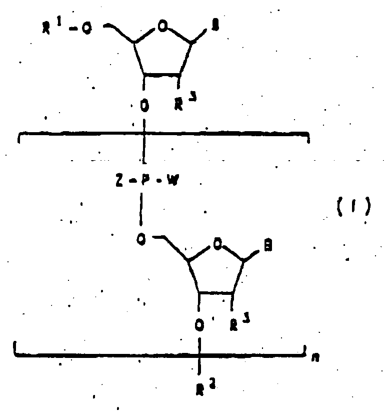 dans laquelle
R¹ représente un atome d'hydrogène ou un radical de formule II 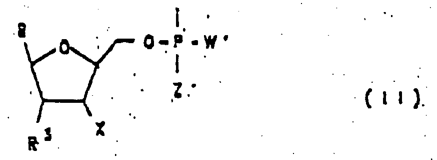
R² représente un radical de formule III 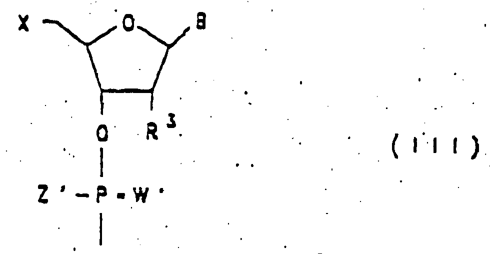
B représente une base, comme par exemple des bases naturelles telles que l'adénine, la thymine, la cytosine, la guanine, ou des bases non naturelles, comme par exemple la purine, la 2,6-diaminopurine, la 7-déazaadénine, la 7-déazaguahine, la N⁴, N⁴-éthanocytosine ou leurs précurseurs ; la base uracil ou cytosine étant contenue dans l'oligoribonucléotide ;
les radicaux R³ représentent, indépendamment les uns des autres, OH, un atome d'hydrogène ou F, et au maximum un radical R³ est égal à H ; et R³ étant F soit pour l'ensemble des nucléotides U ou pour l'ensemble des nucléotides U et C ;
W et W' représentent, indépendamment l'un de l'autre, un atome d'oxygène ou de soufre ;
Z et Z' représentent, indépendamment l'un de l'autre, O⁻, S⁻, un groupe alcoxy en C₁-C₁₈, de préférence un groupe alcoxy en C₁-C₈, un groupe alkyle en C₁-C₁₈, de préférence un groupe alkyle en C₁-C₈, NHR⁴, R⁴ représentant un groupe alkyle en C₁-C₁₈ ou un groupe alcoxy (C₁-C₄)-alkyle-(C₁-C₆), de préférence le groupe méthoxyéthyle ; NR⁴R⁵, R⁴ étant tel que défini ci-dessus
et R⁵ représentant un groupe alkyle en C₁-C₁₈, de préférence un groupe alkyle en C₁-C₈, ou R⁴ et R⁵ formant ensemble, avec l'atome d'azote qui les porte, un cycle hétérocyclique à 5-6 chaînons, qui peut en outre comporter un autre hétéroatome appartenant à la série O, S, N, comme par exemple le cycle morpholine ;
X représente OH, H, F, Cl, Br, NH₂, N₃, un groupe O-C(O)-alkyle(C₁-C₁₈), OC(O)-alcényle(C₂-C₁₈) O-C(O)-alcynyle-(C₂-C₁₈), O-C(O)-aryle(C₆-C₁₈), O-alkyle(C₁-C₁₈), O-alcényle(C₂-C₁₈), O-alcynyle(C₂-C₁₈), o-aryle(C₆-C₁₈), P(O)YY', Y et Y' étant définis comme Z et Z';
dans la formule II, R³ et X peuvent former ensemble un diester d'acide phosphorique cyclique ;
de préférence, X représente OH, H, F ;
n représente un nombre entier allant de 5 à 60, de préférence de 15 à 25,
ainsi que leurs sels physiologiquement acceptables.

2. Oligonucléotide de formule 1 selon la revendication 1, dans lequel R³ est F pour l'ensemble des nucléotides U et C.

3. Oligoribonucléotides de formule I selon la revendication 1 ou la revendication 2, **caractérisés en ce que** R² représente un radical de formule III et R1 représente un atome d'hydrogène ; R¹ et R² représentent un radical de formule II ou III, respectivement, dans ce dernier cas soit W, soit Z ne représentant pas un atome d'oxygène et X représente OH ou H.

4. Oligoribonucléotides de formule I selon l'une des revendications 1 à 3, **caractérisés en ce que** W représente un atome d'oxygène ou Z et W représentent l'un et l'autre un atome d'oxygène.

5. Oligoribonucléotides de formule I selon les revendications 1 à 4, **caractérisés en ce que** R² représente un radical de formule III et R¹ représente un atome d'hydrogène.

6. Oligoribonucléotides de formule I selon les revendications 1 à 5, **caractérisés en ce qu'**ils sont en outre substitués par des groupes qui favorisent le transport intracellulaire, qui servent de groupes « rapporteurs » in vitro ou in vivo et/ou par des groupes qui, lors de l'hybridation de l'oligoribonucléotide avec de l'ADN ou de l'ARN biologique, attaquent par liaison ou coupure ces molécules d'ADN ou d'ARN.

7. Oligoribonucléotides selon la revendication 1, **caractérisés en ce qu'**il en résulte une ribozyme à extrémité(s) 5' et/ou 3' synthétique(s).

8. Procédé pour la préparation des oligoribonucléotides de formule 1, selon les revendications 1 à 6, **caractérisé en ce que**
a) on fait réagir une entité nucléotidique à groupements 3'- ou 5'-terminaux, contenant du phosphore-(III) ou du phosphore-(V), ou un dérivé activé de celle-ci, avec une autre entité nucléotidique à groupe hydroxy 3'- ou 5'-terminal libre, ou
b) on fait la synthèse de la même façon de l'oligonucléotide, au moyen de fragments, on élimine éventuellement, dans les oligonucléotides obtenus selon (a) ou (b), un ou plusieurs groupes protecteurs introduits temporairement pour la protection d'autres fonctions, et les oligonucléotides de formule I obtenus sont éventuellement convertis en un de leurs sels physiologiquement acceptables.

9. Oligoribonucléotides de formule I, selon les revendications 1 à 6, utiles pour des procédés chimiques d'hybridation, destinés à la régulation ou à la répression de la fonction biologique d'acides nucléiques, reposant sur l'appariement à des acides nucléiques mono- ou bicaténaires, ainsi que pour la répression sélective de l'expression de fonctions génomiques virales et pour la prophylaxie et la thérapie de fonctions virales, pour la répression de la fonction oncogène et pour le traitement de maladies cancéreuses.

10. Médicament contenant un ou plusieurs des composés selon les revendications 1 à 7, éventuellement conjointement avec des adjuvants et/ou véhicules physiologiquement acceptables, de préférence pour l'administration intraveineuse ou topique.
